(19) Europäisches Patentamt
European Patent Office
Office européen des brevets

(11) Publication number: 0 121 995
B1

(12) # EUROPEAN PATENT SPECIFICATION

(45) Date of publication of patent specification: 28.09.88

(51) Int. Cl.$^4$: C 07 K 7/08, A 61 K 37/02

(21) Application number: 84301123.0

(22) Date of filing: 22.02.84

(54) **Peptides.**

(30) Priority: 09.03.83 JP 38564/83

(43) Date of publication of application:
17.10.84 Bulletin 84/42

(45) Publication of the grant of the patent:
28.09.88 Bulletin 88/39

(84) Designated Contracting States:
CH DE FR GB LI

(56) References cited:

None

(73) Proprietor: KOWA COMPANY, LTD.
6-29, 3-chome Nishiki
Naka-ku Nagoya-shi Aichi-ken (JP)

(72) Inventor: Aoki, Nobuo
263-59, Yamamoto-cho
Utsunomiya-shi Tochigi-ken (JP)
Inventor: Tamaki, Taro
4-38-7, Tamagawa
Setagaya-ku Tokyo (JP)

(74) Representative: Webb, Frederick Ronald et al
G.F. Redfern & Co. Marlborough Lodge 14
Farncombe Road
Worthing West Sussex BN11 2BT (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to novel peptides.

The inventors of the present invention have previously isolated and purified an antifibrinolytic substance from human plasma and have named the substance "$\alpha_2$-plasmin inhibitor" (which may hereinafter be referred to simple as "$\alpha_2$-PI"). It has been reported by these inventors that $\alpha_2$-PI inhibits plasmin instantaneously and irreversibly and hence inhibits the binding of plasminogen with fribrin, resulting in suppressed activation of the plasminogen on the fibrin and impaired fibrinolysis [see "Prog. Cardiovasc. Dis.", 21, 267-286 (1979)].

It has also been confirmed that when blood coagulation takes place, $\alpha_2$-PI is cross-linked to the $\alpha$-chains of fibrin by the activated blood coagulation factor XIII (an activated fibrin-stabilizing factory), the $\alpha_2$-PI-coupled fibrin is more resistant to physiological fibrinolysis, and $\alpha_2$-PI when coupled with the fibrin serves to act as a glutamine substrate for this factor XIII.

The present inventors have carried out continuous research on substances having an inhibitory effect against the activity of $\alpha_2$-PI. Through research efforts leading to this invention, it has now been found that among peptides composed of amino acids having a structure similar to the N-terminal structure of $\alpha_2$-PI, some selected homologs are capable of inhibiting the activity of $\alpha_2$-PI.

It is accordingly, an object of this invention to provide peptides which are of significant advantage as medicines for fibrinolysis acceleration and also as reagents for the diagnosis of hemostatic disorders, thrombosis and the like.

According to the invention, there is provided a peptide of the following (1):

```
        (CH2)2-COR
         |
Asn-NH-CH-CO-Glu-Gln-Val-Ser-Pro-Leu-Thr-Gly-Leu-Lys-NH2      (I)
 1      2     3   4   5   6   7   8   9   10  11  12
```

where R is OH, an amino or chromophore group.

The peptides falling within the formula (I) can be represented by the formulae (Ia) and (Ib):

```
Asn-Gln-Glu-Gln-Val-Ser-Pro-Leu-Thr-Gly-Leu-Lys-NH2      (Ia)
 1   2   3   4   5   6   7   8   9   10  11  12

        (CH2)2-COR
         |
Asn-NH-CH-CO-Glu-Gln-Val-Ser-Pro-Leu-Thr-GLy-Leu-Lys-NH2      (Ib)
 1            3   4   5   6   7   8   9   10  11  12
```

where R is a chromophore group.

The novel peptides of the formula (I) above have an inhibitory activity effect against the $\alpha_2$-plasmin inhibitor present in blood. Such a compound is a good glutamine substrate for the activated blood coagulation factor XIII and hence is effectively useful as a medicine capable of exhibiting a fibrinolysis-accelerating effect and also as a diagnostic reagent for hemostatic disorders, thrombosis and the like.

A fuller understanding of this invention and many of the attendant advantages thereof will be gathered from the following detailed description when considered in connection with the accompanying drawings, in which:

FIGURE 1 is a diagram illustrating the cross-linking capacity of a peptide according to the invention of the formula (Ia), to fibrin;

FIGURE 2 is a diagram illustrating the inhibitory effect of the peptide of formula (Ia) against the cross-linking of $\alpha_2$-PI to fibrin;

FIGURE 3 is a diagram illustrating the fibrinolysis-accelerating effect of the peptide (Ia); and

FIGURES 4 and 5 are diagrams illustrating the synergistic effects accruing from the combined use of the peptide (Ia) and a tissue plasminogen activator.

A peptide of the formula (I) according to this invention may be prepared by a variety of processes which are commonly employed for peptide synthesis. In general, a solid-phase or liquid-phase process may be used which makes it possible to apply, in combination, a method involving successive coupling of component amino acids, a fragment condensation method and the like.

Where component amino acids contain other functional groups, suitable protecting groups are used to protect these functional groups. The connection and subsequent removal of the protecting groups may be carried out by combining suitable procedures in various ways.

More specifically, the peptide (I) of the invention may be produced, for example, through successive condensations, beginning with the lysine (No. 12) of the formula (I) by a solid-phase process using a polystyrenebenzhydrylamine resine.

As an $\alpha$-amino protecting group, use may be made of a t-butyloxycarbonyl group. Suitable protecting groups for the functional groups of amino acids on their side chains include an o-chlorobenzyloxycarbonyl group for the amino group, and a benzyl group for the hydroxyl group, and a benzyl ester group for the carboxyl group.

For releasing the peptide (I) from the resin, hydrogen fluoride may be used to treat the peptide-carrying resin at low temperatures. In this way, the peptide (I) is readily released and isolated from the resin.

The peptide (I) of the invention is cross-linkable in nature with fibrin in clots present in blood by the activated blood coagulation factory XIII and hence is competitively inhibitory against the cross-linking of $\alpha_2$-PI to the fibrin in the clots. Because of its fibrinolysis-accelerating effect, the peptide (1) can therefore be satisfactorily used for pharmaceutical purposes, for example, as a therapeutic drug for thrombosis, either alone, or in combination with other drugs.

Moreover, since the peptide (I) of the invention is a glutamine substrate susceptible to a specific reaction with the activated blood coagulation factor XIII, two compounds derived from the peptide (I) are effectively useful as diagnostic and analytical reagents. One of these compounds is a compound obtained by labelling a peptide of the formula (Ia) with a radioactive element, and the other is a peptide of the formula (Ib) containing a chromophore introduced in the γ-carboxyl group of glutamic acid indicated by the reference numeral 2 in the formula (Ib).

For introducing the chromophore residual group, the various methods stated above may be applied by using a glutamic acid derivative in which a chromophore has been introduced in advance. Alternatively, such a chromophore may be introduced directly into the peptide (Ia).

Any chromophore residual groups may be used, so long as they can be measured in the visible and/or ultraviolet ranges. Suitable examples include p-nitroaniline, and aminomethylcoumarinic acid.

This invention will now be further described with reference to the following experiments and specific examples which are provided for purposes of illustration only and are not to be construed as limiting the invention.

The activities and effects of the peptide (Ia) of the invention were tested with the results given below.

Experiment 1

Cross-linking capacity of the peptide (Ia) with fibrin:

To a mixed solution of 20 μl of the peptide (Ia) labelled with a radioactive $^{125}I$, 20 μl of fibrinogen (30 mg/ml, 88 μM, containing 2 or 10 u/ml of the blood coagulation factor XIII) and 140 μl of a Tris buffer, was added 20 μl of a mixed solution of thrombin (3.3. u/ml) and calcium chloride (50 mM). The resulting mixture was then incubated at 37°C.

The clotting sample was frozen instantaneously at each time with dry ice/acetone to terminate the reaction and then lyophilized. The resulting fibrin film was washed three times, each for 5 minutes, with 200 μl of 50 MM EDTA cntaining 1% of BSA (bovine serum albumin). The fibrin film thus washed was measured with respect to its radioactivity to determine the amount of peptide coupled with the fibrin [see "Biochim, Biophys, Acta", 661, 280—286, (1981)]. The results are shown in Figure 1.

The peptide (Ia) of the invention was crosslinked promptly to the fibrin and reached equilibrium when about 40% of the petide (Ia) had been coupled. The cross-linking velocity of the peptide (Ia) was substantially the same as that of $\alpha_2$-PI.

It has thus been confirmed from these results that the peptide of the invention is a good glutamine substrate for the activated blood coagulation factor XIII.

Experiment 2

Inhibitory effect of the peptide (Ia) against the cross-linking of $\alpha_2$-PI to fibrin:

Twenty microliters of a mixed solution of thrombin (3.3 u/ml) and calcium chloride (50 mM) was added to a mixed solution consisting of 20 μl of fibrinogen (30 mg/ml, 88 μM, containing 10 u/ml of the blood coagulation factor XIII), 20 μl of $\alpha_2$-PI (0.67 mg/ml, 10 μM), 20 μl of a $^{125}I$-labelled peptide (0—50 mM) and 120 μl of a Tris buffer. The amount of the peptide bonded to the resulting fibrin clot was then determined in the same manner as in Experiment 1.

On the other hand, the amount of $\alpha_2$-PI coupled with a fibrin clot was also determined using $^{125}I$-labelled $\alpha_2$-PI and an unlabelled peptide. The results are shown in Figure 2.

From the above results, it will be appreciated that the peptide of the invention inhibits competitively the cross-linking of $\alpha_2$-PI to the fibrin.

Experiment 3

Fribrinolysis-accelerating effect of the peptide (Ia):

Healthy human blood, to which sodium citrate had been added, was subjected to centrifugal separation to prepare platelet-rich plasma. A trace amount of $^{125}I$-labelled fibrinogen was added to and mixed with the platelet-rich plasma. 250 mM solutions of calcium chloride which contained the peptide (Ia) in different amounts (0—5 mM) were poured into glass test tubes, each in a volume of 20 μl, 180 μl of the above plasma was added to each test tube and then allowed to stand at 37°C. A fibrin clot was formed in a few minutes. Clot retraction started about 15 minutes later and was completed in about 30 minutes.

Thereafter, 800 μl of platelet-poor plasma which contained the peptide (Ia) in different amounts (0—500 μM) and 1 u of Hirudin was added to each of the test tubes. Each test tube was then incubated at 37°C. The contents were sampled periodically to measure $^{125}I$ released into the supernatant liquid. The results are shown in Figure 3.

It is believed that the peptide (Ia) of the invention has a fibrinolysis-accelerating effect because the

dissolution of each fibrin clot is promoted by addition of the peptide (Ia), as compared with the case where the peptide (Ia) is absent and that this effect is proportional to the amount of the peptide (Ia) added.

Experiment 4

Synergistic effects of the peptide (Ia) used in combination with a plasminogen activator:

*(1) Addition of the peptide (Ia) and the activator before clot formation:*

Healthy human blood to which sodium citrate had been added, was subjected to centrifugal separation to prepare platelet-rich plasma. A trace amount of $^{125}$I-labelled fibrinogen was added to and mixed with the platelet-rich plasma. 250 mM solutions of calcium chloride which contained the peptide (Ia) in different amounts (0—5 mM) and 1 u/ml of a melanoma-derived tissue plasminogen activator (TPA), were poured into glass test tubes, 20 µl by 20 µl to each of which was then added 180 µl of the above plasma. A fibrin clot was formed in a few minutes. Clot retraction started about 15 minutes later and was completed in about 30 minutes.

Thereafter, to each test tube was added 800 µl of a platelet-poor plasma which contained the peptide (Ia) in different amounts (0—500 µM) and 1 u of Hirudin. The test tubes were then incubated at 37°C. The contents were sampled periodically to measure $^{125}$I released into the supernatant liquid. The results are shown in Figure 4.

*(2) Addition of the peptide (Ia) and the activator after completion of clot formation*

Healthy human blood to which sodium citrate had been added, was subjected to centrifugal separation to prepare platelet-rich plasma. A trace amount of $^{125}$I-labelled fibrinogen was added to and mixed with the platelet-rich plasma. 250 mM solutions of calcium chloride were poured into glass test tubes, 20 µl by 20 µl, to each of which was then added 180 µl of the above plasma. A fibrin clot was formed in a few minutes. Clot retraction started about 15 minutes later and was completed in about 30 minutes.

Thereafter, to each test tube was added 800 µl of platelet-poor plasma which contained the peptide (Ia) in different amounts (0—500 µM), 1 u of Hirudin and 1.25 u of a melanoma-derived tissue plasminogen activator (TPA). The test tubes were then incubated at 37°C. The contents were sampled periodically to measure $^{125}$I release into the supernatant liquid. The results are shown in Figure 5.

It is apparent that, when used in combination with a plasminogen activator, the peptide (Ia) of the invention brings about a synergistically-enhanced fibrinolysis-accelerating effect, because such effect is substantially increased by adding the tissue plasminogen activator to the peptide (Ia), as compared with the case where no such activator is added.

Example 1

(1) In a manual solid-phase synthesis reactor was placed 1.64 g of a benzhydrylamine resin hydrochloride (which contained 1.0 mmol of amino groups). After being neutralized with a 5% solution of diisopropylethylamine in methylene chloride, the hydrochloride was successively condensed with aminoacids, beginning with the carboxyl-terminated amino acid, by a solid-phase synthesis reaction.

The following amino acids were used after introducing protecting groups wherever needed:—

| | | |
|---|---|---|
| 1) | Boc-L-Lys(o-ClZ) t-butNH$^2$ (used after removal of t-ButNH$_2$) | 1.63 g |
| 2) | Boc-L-Leu $H_2O$ (used after dehydration) | 0.75 g |
| 3) | Boc-L-Gly | 0.53 g |
| 4) | Boc-L-Thr(Bzl) | 0.93 g |
| 5) | Boc-L-Pro | 0.65 g |
| 6) | Boc-L-Ser (Bzl) | 0.89 g |
| 7) | Boc-L-Val | 0.65 g |
| 8) | Boc-L-Gln | 0.74 g |
| 9) | Boc-L-Glu (OBzl) | 1.01 g |
| 10) | Boc-L-Asn | 0.70 g |

(o-ClZ : o-chlorobenzyloxycarbonyl
Bzl : benzyl
OBzl : benzyl ester)

The removal of the t-butyloxycarbonyl group (Boc) was effected by treating each amino acid with a 50% solution of trifluoroacetic acid in methylene chloride at room temperature for 30 minutes.

The neutralization of trifluoroacetic acid was effected by treating the resulting solution with a 5% solution of diisopropylethylamine in methylene chloride for 10 minutes.

The extension of the peptide chain was effected by reacting 3 equivalents of the Boc-amino acid with a methylene chloride solution of dicyclohexylcarbodiimide at room temperature for 3 hours. N-hydroxybenztriazole was added when condensing glutamine and asparagine.

Any excess reagent or reagents in each reaction were removed by washing with methylene chloride, dimethylformamide, isopropyl alcohol, or the like.

By the above-mentioned treatments, 3.3 g of a resin was obtained which contained a peptide having the following amino acid composition:—

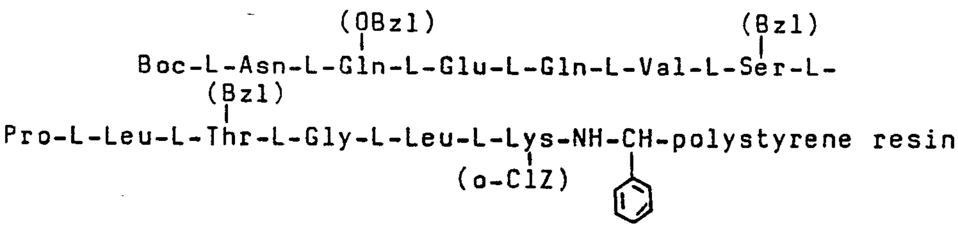

(2) In an anhydrous hydrogen fluoride reactor was placed 2.0 g of the peptide-containing resin obtained in step (1) above. To the resin was added 2 ml of anisole and then 20 ml of hydrogen fluoride. The resulting mixture was reacted at 0°C for 60 minutes.

Excess hydrogen fluoride was evaporated under reduced pressure, and the residue was dissolved in distilled water. The resulting solution was caused to pass through a column packed with "Dowex" X—1 (an acetic acid type, "Dowex" being a trademark, of Dow Chemical Co.), and the effluent was collected and then lyophilized to obtain 623 mg of a crude product. The crude product was dissolved in distillsed water and then subjected to reverse-phase, high-performance liquid chromatogrpahy using an acetonitrile-phosphoric acid buffer as an eluant. The main peak was collected. Acetonitrile was removed under reduced pressure, and the resulting solution was again caused to pass through a column packed with "Dowex" X—1 (an acetic acid type). The effluent was desalted and then lyophilized to obtain 130 mg of the following intended peptide:—

L-Asn-L-Gln-L-Glu-L-Gln-L-Val-L-Ser-L-Pro-

L-Leu-L-Thr-L-Gly-L-Leu-L-Lys-$NH_2$

| | |
|---|---|
| Appearance: | Colourless powder |
| Rotary Power: | $[\alpha]_D^{32}$ − 96.5 (c 0.26, $H_2O$) |
| Elemental analysis (%) | Calculated for<br>$C_{56}H_{97}N_{17}O_{19}$ $CH_3COOH$ $10H_2O$:<br>C, 44.86; H, 7.86; N, 15.34<br>Found:<br>C, 44.87; H, 7.24; N, 15.67 |
| Amino acid analysis: | 6N HCl, 110°C, 42-hour phenol treatment<br>Lys(1) 1.02, $NH_3$(4) 5.20,<br>Asp(1) 0.98, Thr(1) 0.95,<br>Ser(1) 0.87, Glu(3) 2.91,<br>Pro(1) 1.01, Gly(1) 1.00,<br>Val(1) 0.99, Leu(2) 1.98 |
| Silica gel thin-layer chromatography: | Developing solvent (n-butyl alcohol : acetic acid : water : pyridine = 15 : 3 : 12 : 10) $R_f$ value: 0.55 (single spot) |

Example 2

The procedure of Example 1 was repeated, except that

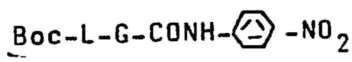

was used in place of

Boc-L-Gln,

to obtian the following compound:—

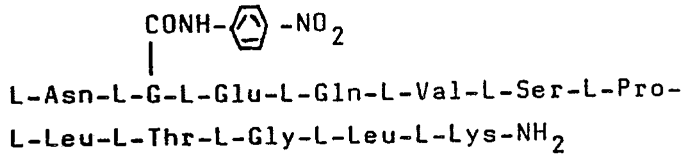

## Claims

1. A peptide of the formula (I):

(CH2)2-COR
Asn-NH-CH-CO-Glu-Gln-Val-Ser-Pro-Leu-Thr-Gly-Leu-Lys-NH2 (I)
1 2 3 4 5 6 7 8 9 10 11 12

where R is OH, an amino or chromophore group.

2. The peptide as claimed in Claim 1 having the formula (Ia):—

Asn-Gln-Glu-Gln-Val-Ser-Pro-Leu-Thr-Gly-Leu-Lys-NH2 (Ia)
1 2 3 4 5 6 7 8 9 10 11 12

3. A peptide as claimed in Claim 1 having the formula (Ib):—

(CH2)2-COR
Asn-NH-CH-CO-Glu-Gln-Val-Ser-Pro-Leu-Thr-Gly-Leu-Lys-NH2 (Ib)
1 2 3 4 5 6 7 8 9 10 11 12

where R is a chromophore group.

4. The use of a peptide as claimed in any one of Claims 1 to 3 as a therapeutic drug for the treatment of thrombosis and hemostatic disorders.

5. The use of a peptide as claimed in any one of Claims 1 to 3 as a diagnostic reagent for hemostatic disorders and thrombosis.

## Patentansprüche

1. Peptid der Formel (I):

(CH2)2-COR
Asn-NH-CH-CO-Glu-Gln-Val-Ser-Pro-Leu-Thr-Gly-Leu-Lys-NH2 (I)
1 2 3 4 5 6 7 8 9 10 11 12

wobei R OH, eine Aminogruppe oder chromophore Gruppe ist.

2. Peptid nach Anspruch 1 mit der Formel (Ia):

Asn-Gln-Glu-Gln-Val-Ser-Pro-Leu-Thr-Gly-Leu-Lys-NH2 (Ia)
1 2 3 4 5 6 7 8 9 10 11 12

3. Peptid nach Anspruch 1 mit der Formel (Ib):

(CH2)2-COR
Asn-NH-CH-CO-Glu-Gln-Val-Ser-Pro-Leu-Thr-Gly-Leu-Lys-NH2 (Ib)
1 2 3 4 5 6 7 8 9 10 11 12

wobei R eine chromophore Gruppe ist.

4. Verwendung eines Peptids nach einem der Ansprüche 1 bis 3 als Therapeutikum zur Behandlung von Thrombosen und hämostatischen Störungen.

5. Verwendung eines Peptids nach einem der Ansprüche 1 bis 3 als Diagnostikum für hämostatische Störungen und Thrombosen.

**Revendications**

1. Peptide de formule (I):

$$\begin{array}{l} \quad\quad\quad (CH_2)_2-COR \\ \quad\quad\quad | \\ Asn-NH-CH-CO-Glu-Gln-Val-Ser-Pro-Leu-Thr-Gly-Leu-Lys-NH_2 \end{array} \quad (I)$$

1 2 3 4 5 6 7 8 9 10 11 12

dans laquelle R est OH, un groupe amino ou un groupe chromophore.

2. Peptide selon la revendication 1 de formule (Ia):

Asn-Gln-Glu-Gln-Val-Ser-Pro-Leu-Thr-Gly-Leu-Lys-NH2 (Ia)

1 2 3 4 5 6 7 8 9 10 11 12

3. Peptide selon la revendication 1 de formule (Ib):

$$\begin{array}{l} \quad\quad\quad (CH_2)_2-COR \\ \quad\quad\quad | \\ Asn-NH-CH-CO-Glu-Gln-Val-Ser-Pro-Leu-Thr-GLy-Leu-Lys-NH_2 \end{array} \quad (Ib)$$

1 2 3 4 5 6 7 8 9 10 11 12

dans laquelle R est un groupe chromophore.

4. Utilisation d'un peptide selon l'une quelconque des revendications 1 à 3, comme médicament pour le traitement des thromboses et des désordres hémostatiques.

5. Utilisation d'un peptide selon l'une quelconque des revendications 1 à 3, comme agent de diagnostic pour les désordres hémostatiques et les thromboses.

FIG.1

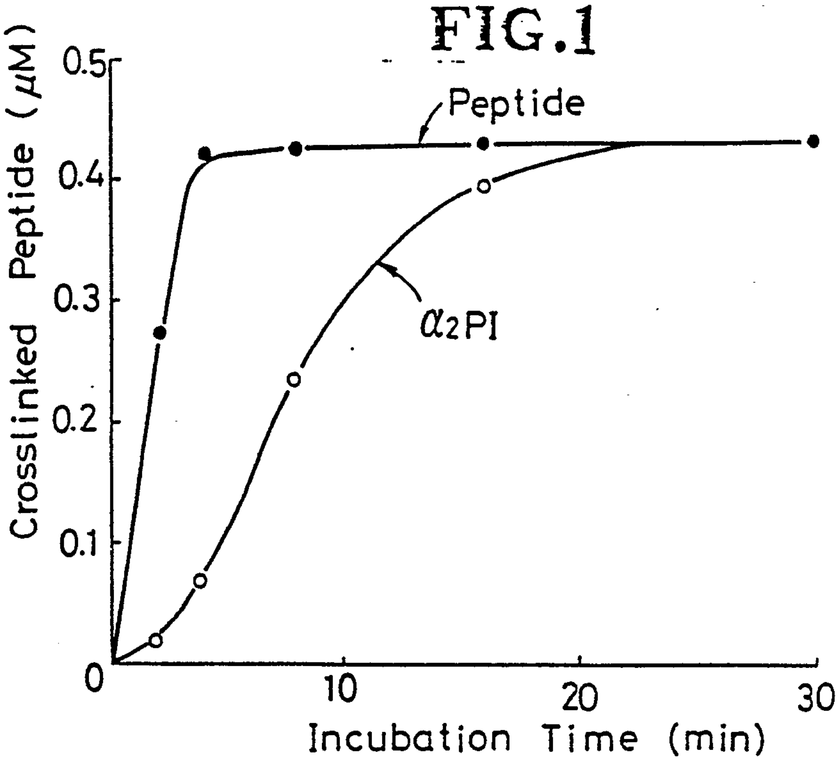
Crosslinked Peptide (μM)
0.5
0.4
0.3
0.2
0.1
0
Peptide
α2PI
10
20
30
Incubation Time (min)

FIG.2

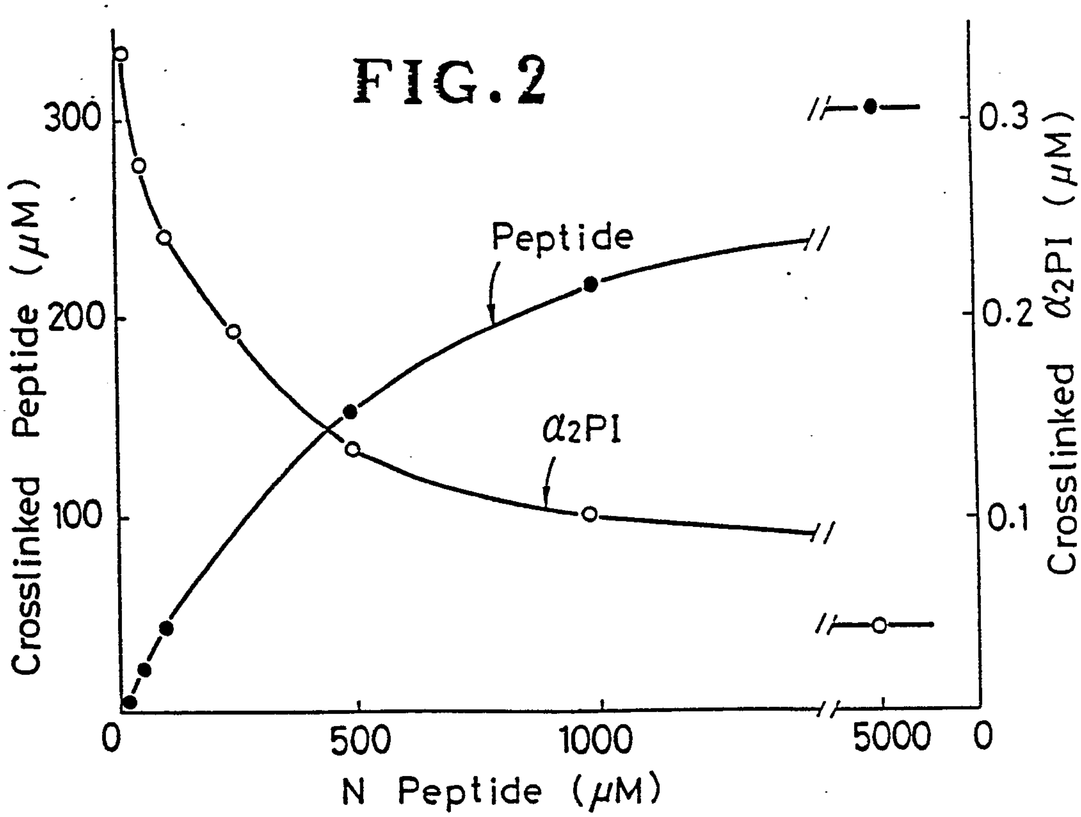
Crosslinked Peptide (μM)
300
200
100
Peptide
α2PI
0
500
1000
5000
0
N Peptide (μM)
0.3
0.2
0.1
Crosslinked α2PI (μM)

# FIG. 3

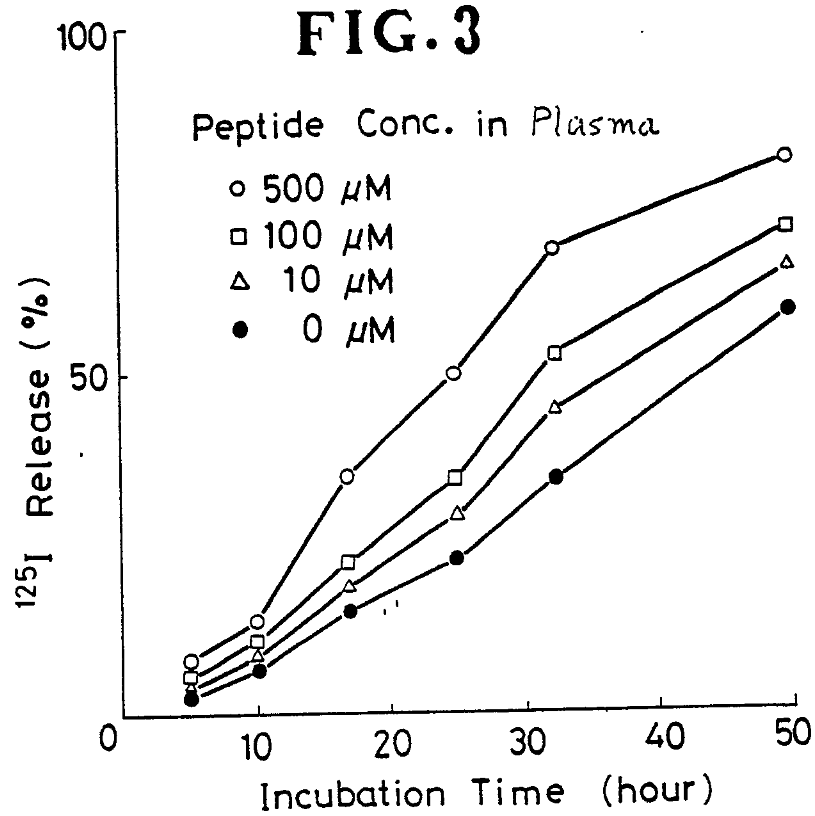
100
50
0
$^{125}$I Release (%)
Peptide Conc. in Plasma
500 μM
100 μM
10 μM
0 μM
10
20
30
40
50
Incubation Time (hour)

# FIG. 4

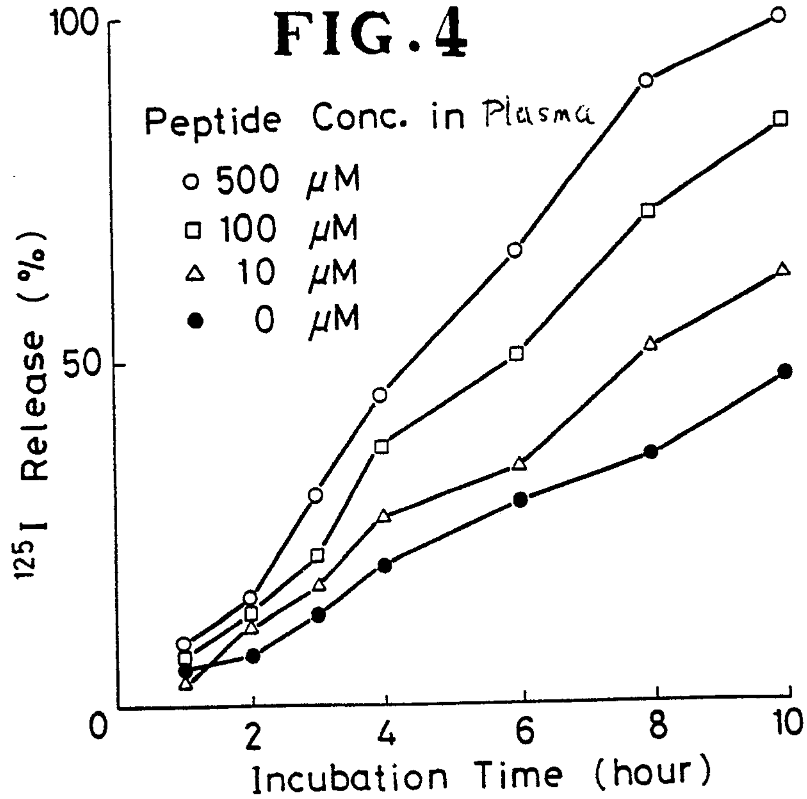
100
50
0
$^{125}$I Release (%)
Peptide Conc. in Plasma
500 μM
100 μM
10 μM
0 μM
2
4
6
8
10
Incubation Time (hour)

# FIG.5

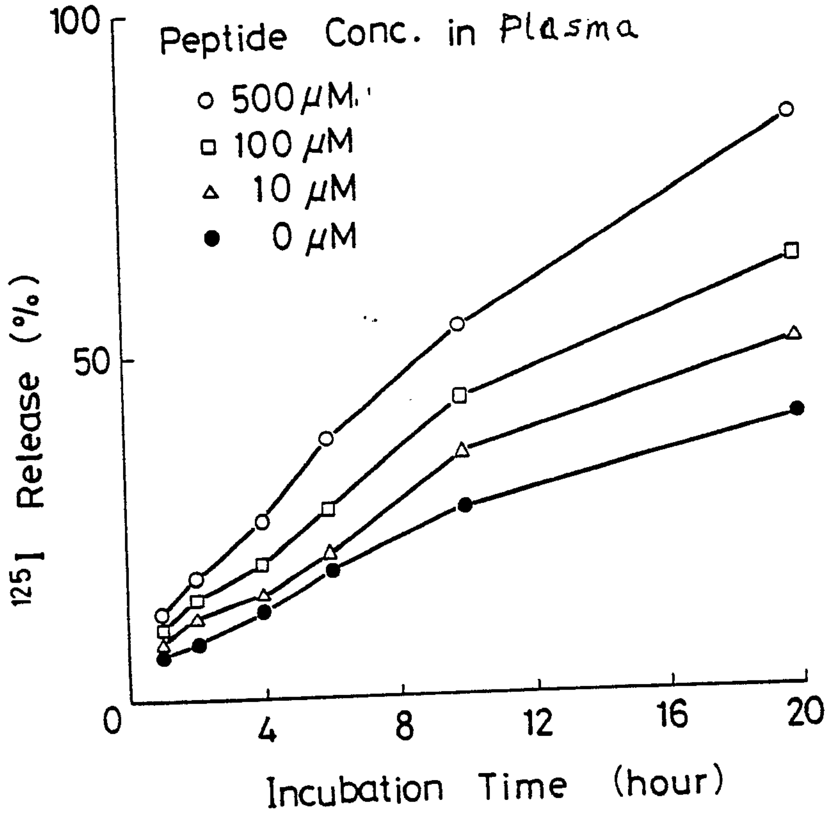
Peptide Conc. in Plasma
500 μM
100 μM
10 μM
0 μM
100
50
0
$^{125}$I Release (%)
4
8
12
16
20
Incubation Time (hour)